(19) Europäisches Patentamt / European Patent Office / Office européen des brevets

(11) **EP 2 217 303 B1**

(12) **EUROPÄISCHE PATENTSCHRIFT**

(45) Veröffentlichungstag und Bekanntmachung des Hinweises auf die Patenterteilung:
**23.03.2016 Patentblatt 2016/12**

(51) Int Cl.:
*A61M 1/16* [(2006.01)]   *A61M 1/36* [(2006.01)]

(21) Anmeldenummer: **08853089.4**

(22) Anmeldetag: **22.11.2008**

(86) Internationale Anmeldenummer:
**PCT/EP2008/009917**

(87) Internationale Veröffentlichungsnummer:
**WO 2009/065611 (28.05.2009 Gazette 2009/22)**

(54) **VORRICHTUNG ZUM BESTIMMEN DER REZIRKULATION IN EINER FISTEL ODER DER KARDIOPULMONALEN REZIRKULATION SOWIE BLUTBEHANDLUNGSVORRICHTUNG MIT EINER VORRICHTUNG ZUR BESTIMMUNG DER FISTELREZIRKULATION ODER DES KARDIOPULMONALEN REZIRKULATIONSANTEILS**

DEVICE FOR DETERMINING THE RECIRCULATION IN A FISTULA OR THE CARDIOPULMONARY RECIRCULATION, AND A BLOOD TREATMENT DEVICE COMPRISING A DEVICE FOR DETERMINING THE FISTULA RECIRCULATION OR THE CARDIOPULMONARY RECIRCULATION PART

DISPOSITIF POUR DÉTERMINER LA RECIRCULATION DANS UNE FISTULE OU LA RECIRCULATION CARDIOPULMONAIRE, ET DISPOSITIF DE TRAITEMENT DU SANG POURVU D'UN DISPOSITIF POUR DÉTERMINER LA CIRCULATION EN FISTULE OU LA PART DE RECIRCULATION CARDIOPULMONAIRE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **22.11.2007 DE 102007056475**

(43) Veröffentlichungstag der Anmeldung:
**18.08.2010 Patentblatt 2010/33**

(73) Patentinhaber: **Fresenius Medical Care Deutschland GmbH**
**61352 Bad Homburg v.d.H. (DE)**

(72) Erfinder:
• **ZHANG, Wei**
  **97464 Niederwerrn (DE)**

• **SCHULTE, Elke**
  **97422 Schweinfurt (DE)**
• **BARDORZ, Christoph**
  **97228 Rottendorf (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**WO-A-2006/072271    DE-C1- 19 528 907**
**DE-C1- 19 541 783**

**Beschreibung**

Die Erfindung betrifft eine Vorrichtung zum Bestimmen der Rezirkulation in einer Fistel und/oder des kardiopulmonalen Rezirkulationsanteils während einer extrakorporalen Blutbehandlung, bei der das zu behandelnde Blut in einem extrakorporalen Blutkreislauf durch eine Blutkammer eines durch eine semipermeable Membran in die Blutkammer und eine Dialysierflüssigkeitskammer unterteilten Dialysators strömt und Dialysierflüssigkeit in einem Dialysierflüssigkeitsweg durch die Dialysierflüssigkeitskammer des Dialysators strömt, Darüber hinaus bezieht sich die Erfindung auf eine Blutbehandlungsvorrichtung mit einer Vorrichtung zum Bestimmen der Fistetrezirkulation und/oder des kardiopulmonalen Rezirkulationsanteils.

Bei Verfahren der chronischen Blutreinigungstherapie, wie Hämodialyse, Härnofiltration und Härnodiafiltration, wird Blut über einen extrakorporalen Blutkreislauf geleitet. Als Zugang zurn Blutgefäßsystem wird häufig operativ eine arteriovenöse Fistel angelegt. Ebenso ist der Einsatz eines Implantats möglich. Wenn nachfolgend von einer "Fistel" die Rede ist, wird darunter jede Art der Verbindung zwischen einer Vene und einer Arterie des Patienten verstanden.

Das durch die Fistel fließende Blut wird nur während der eigentlichen Dialysebehandlung benutzt. Im dialysefreien Zeitraum entspricht der Blutfluss in der Fistel einem funktionellen Links/Rechts-Shunt, bei dem ein Anteil des arteriellen Blutes aus dem Herzminutenvolumen (HMV) unter Umgehung einer peripheren Nutzung direkt dem venösen System und dem Herzen zugeführt wird. Der Fistelfluss rezirkuliert über Herz und Lungen. Der fraktionelle Anteil des Fistelflusses am Herzminutenvolumen wird als kardiopulmonare Rezirkulation definiert. Die kardiopulmonare Rezirkulation hat nicht nur Auswirkungen auf die Kreislaufbelastung des Patienten, sondern auch auf die Effizienz der Dialyse. Da das dialysierte Blut aus dem extrakorporalen Kreislauf unter Umgehung der systemischen Kreislaufgebiete dem venösen Rückstrom aus dem großen Körperkreislauf beigemischt wird, kommt es zu einer systematischen Reduktion in der Konzentration dialysierbarer Bestandteile im arteriellen Blut (D. Schneditz et al.: Cardiopulmonary recirculation during hemodialysis. Kidney Int. 42: 1450 -1456, 1992).

Für die Funktionsfähigkeit der Fistel ist deren Perfusion von Bedeutung. Sinkt der Fistelfluss unter einen kritischen Wert, dann steigt das Risiko einer Fistelthrombose mit dem möglichen Verlust des Gefäßzuganges, was in der Dialysebehandlung eine erhebliche Komplikation darstellt (W. Bay et al.: Color Doppler flow predicts PTFE graft failure, J. Am. Soc. Nephrol. 5: 407 (1994)). Ist der Fistelfluss während der Dialysebehandlung kleiner als der extrakorporale Blutfluss ($Q_B$), kommt es zur lokalen Fistelrezirkulation, wobei eine Fraktion des dialysierten und mit der venösen Blutleitung zur Fistel zurückgeführten Bluts über die arterielle Blutleitung dem Dialysator wieder zugeführt wird. Die Fistelrezirkulation ($R_A$) bewirkt eine bedeutende Verminderung der Dialyseeffizienz (F. Gotch: "Models to predict recirculation and its effect on treatment time in single-needle dialysis" First Intl. Symposium on Single-Needle Dialysis, Hrsg.: S. Rignoir. R. Vanholder und P. Invanovich, Cleveland, ISAO Press, 1984, Seite 305 ff.). Die Messung der Qualität des Gefäßzuganges ist somit ein wichtiges Mittel zur Qualitätssicherung bei der Dialysebehandlung.

Aufgrund ihrer klinischen Bedeutung sind verschiedene Verfahren zur Messung der Rezirkulation bekannt. Allen gemeinsam ist die Messung einer physikalischen oder chemischen Kenngröße des Blutes, die im venösen Zweig des extrakorporalen Kreislaufs verändert wird. Die physikalische oder chemische Kenngröße des Blutes kann durch eine manuelle Injektion eines Indikators oder auch mittelbar über die Dialyseaufbereitungseinheit geändert werden.

Wenn nachfolgend von einer Rezirkulation ($R$), einer Fistelrezirkulation ($R_A$) und einem kardiopulmonalen Rezirkulationsanteil ($R_{CP}$) die Rede ist, werden unter diesen Begriffen nicht absolute Größen, sondern der Anteil der jeweiligen Rezirkulation am Herzminutenvolumen verstanden. In der Praxis sind relative Größen ausreichend, um die Rezirkulationsvorgänge in der Fistel beurteilen zu können.

Aus EDTNA-ERCA Journal 19, 6 (1993) ist ein als Thermodilution bezeichnetes Verfahren zur Rezirkulationsmessung bekannt. Bei dem bekannten Verfahren wird ein kurzzeitiger Temperaturabfall im Dialysierflüssigkeitskreislauf iniziert, der sich auf den venösen Zweig des extrakorporalen Kreislaufs überträgt und zu einem nachweisbaren Temperatursprung im arteriellen Zweig des extrakorporalen Kreislaufs dann führt, wenn eine Rezirkulation auftritt.

Eine bekannte Vorrichtung zur Durchführung des als Thermodilution bezeichneten Verfahrens weist einen im arteriellen Zweig und einen im venösen Zweig des extrakorporalen Kreislaufs angeordneten Temperaturfühler auf. Mit dem venösen Temperaturfühler wird der Temperatursprung aufgenommen, der auf den im Dialysierflüssigkeitskreislauf erzeugten Temperaturabfall zurückzuführen ist. Der gemessene Temperatursprung wird ausgewertet und nachfolgend mit dem im arteriellen Messfühler registrierten Temperaturverlauf verglichen. Das Verhältnis der beiden TemperaturIntegrale zueinander oder das Amplitudenverhältnis ist ein Maß für die gesamte Effizienzminderung der Dialysebehandlung durch fistel- und kardiopulmonale Rezirkulation.

Die bekannte Vorrichtung zur Rezirkulationsmessung hat sich in der Praxis bewährt. Als nachteilig erweist sich jedoch, dass nur die nachfolgend als Rezirkulation ($R$) bezeichnete Gesamtrezirkulation gemessen werden kann, die der Summe der Fistelrezirkulation ($R_A$) und einem auf die kardiopulmonale Rezirkulation zurückgehenden Anteil, im Folgenden als kardiopulmonaler Rezirkulationsanteil ($R_{CP}$) bezeichnet, entspricht. Der kardiopulmonale Rezirkulationsanteil ($R_{CP}$) muss hierbei von dem Fistelflussanteil am Herzminutenvolumen, im Folgenden als kardiopulmonale Rezirkulation ($R_{Cp}$) bezeichnet, unterschieden werden.

Das als Thermodilution bezeichnete Verfahren zur Messung der Gesamt-Rezirkulation, die sich aus Fistel- und kardiopulomaler Rezirkulation zusammensetzt, ist auch in DRUKKER; PARSONS and MAHER, Replacement of Renal Function by Dialysis, 5th Edition, 2004, Kluwer Academic Publishers BV beschrieben.

Ein Verfahren zur Messung der Rezirkulation (R), d.h. der Summe der Fistelrezirkulation ($R_A$) und des kardiopulmonalen Rezirkulationsanteils ($R_{CP}$), ist auch aus der DE 197 02 441 C1 bekannt. Bei dem bekannten Verfahren wird eine physikalische oder chemische Kenngröße der Dialysierflüssigkeit im Dialysierflüssigkeitsweg stromauf des Dialysators verändert, die zu einer Änderung der physikalischen oder chemischen Kenngröße auf der Blutseite führt. Die Änderung der Kenngröße der Dialysierflüssigkeit auf der Blutseite führt zu einer Änderung der Kenngröße der Dialysierflüssigkeit stromab der Dialysierflüssigkeitskammer des Dialysators. Zur Bestimmung der Rezirkulation wird die Kenngröße im Dialysierflüssigkeitsweg stromab des Dialysators gemessen und aus dem zeitlichen Verlauf der Änderung der Kenngröße wird die Rezirkulation (R) bestimmt. Als physikalische oder chemische Kenngröße kann die Dialysierflüssigkeits-IonenKonzentration, beispielsweise die Na-Konzentration der Dialysierflüssigkeit, oder auch die Temperatur der Dialysierflüssigkeit verändert und gemessen werden. Nachteilig ist jedoch wieder, dass mit dem bekannten Verfahren nicht die Fistelrezirkulation oder kardiopulmonale Rezirkulation, sondern nur die gesamte Rezirkulation bestimmt werden kann.

Die DE-A-195 28 907 C1 beschreibt ein Verfahren zur Bestimmung der kardiopulmonalen Rezirkulation. Die Messung der kardiopulmonalen Rezirkulation beruht auf zwei kurz aufeinander folgenden Messungen der Rezirkulationsfraktion, die automatisch vor und nach der Umkehrung des Blutflusses durchgeführt werden. Nachteilig ist, dass das bekannte Verfahren die Umkehr des Blutflusses erfordert.

Die US 6 537 240 B2 beschreibt ein Verfahren zur Bestimmung der Rezirkulation, bei dem die Ultrafiltrationsrate verändert wird und vor und nach der Änderung der Ultrafiltrationsrate ein Wert eines Blutparameters bestimmt wird, der für das Verhältnis des Blutplasmavolumens zu dem Blutvolumen repräsentativ ist.

Die WO2006/072271 A1 beschreibt eine Vorrichtung zur Erkennung von Komplikationen während einer extrakorporalen Blutbehandlung, die auf der Überwachung der Dialysance auf der Grundlage von dialysatseitigen Messgrößen und der Überwachung der Rezirkulation auf der Grundlage von blutseitigen Messgrößen beruht. Bei der bekannten Vorrichtung wird aber nur die Gesamtrezirkulation bestimmt, die sich aus Fistel- und kardiopulmonaler Rezirkulation zusammensetzt.

Die DE 195 41 783 C1 beschreibt eine Vorrichtung zur Bestimmung hämodynamischer Parameter während einer extrakorporalen Blutbehandlung, bei der die Temperatur im arteriellen Zweig des extrakorporalen Kreislaufs unter Variation des Blutflusses gemessen wird, wobei die Temperatur des venösen Zweigs konstant gehalten wird.

Aus der DE 195 28 907 C1 ist eine Vorrichtung zur Bestimmung hämodynamischer Parameter bekannt, die auf zwei kurz aufeinander folgende Messungen der Rezirkulationsfraktion vor und nach Umkehr des Blutflusses beruht.

Der Erfindung liegt die Aufgabe zu Grunde, eine Vorrichtung anzugeben, die die Bestimmung der Fistelrezirkulation und/oder der kardiopulmonalen Rezirkulation während einer extrakorporalen Blutbehandlung ohne die Umkehr des Blutflusses im extrakorporalen Blutkreislauf erlaubt.

Eine weitere Aufgabe der Erfindung ist, eine Vorrichtung zum Bestimmen der Fistelrezirkulation und/oder der kardiopulmonalen Rezirkulation ohne Umkehr des Blutflusses zu schaffen.

Darüber hinaus ist eine Aufgabe der Erfindung, eine Blutbehandlungsvorrichtung bereit zu stellen, die ohne Umkehr des Blutflusses die Bestimmung der Fistelrezirkulation und/oder des kardiopulmonalen Rezirkulationsanteils erlaubt.

Die Lösung dieser Aufgaben erfolgt erfindungsgemäß mit den Merkmalen der Patentansprüche 1 und 8. Vorteilhafte Ausführungsformen der Erfindung sind Gegenstand der Unteransprüche.

Die erfindungsgemäße Vorrichtung beruht darauf, dass die Summe von Fistelrezirkulation ($R_A$) und kardiopulmonalem Rezirkulationsanteil ($R_{CP}$), d.h. die Rezirkulation (R), für zwei verschiedene Blutflussraten bestimmt wird, die sich voneinander unterscheiden. Die Fistelrezirkulation und/oder kardiopulmonale Rezirkulation wird dann aus der Rezirkulation für beide Blutflussraten bestimmt.

Für die erfindungsgemäße Vorrichtung ist unerheblich, nach welchem Verfahren und mit welcher Vorrichtung die Summe von Fistelrezirkulation und kardiopulmonalem Rezirkulationsanteil bestimmt wird. Daher kann die Rezirkulation für die beiden Blutflussraten nach den bekannten Verfahren und mit den bekannten Vorrichtungen bestimmt werden.

Von Vorteil ist, wenn die Messungen zur Bestimmung der Rezirkulation für die anschließende Berechnung der Fistelrezirkulation und kardiopulmonalen Rezirkulation nicht invasiv erfolgen. Daher bietet sich das als Thermodilution bekannte Verfahren zur Rezirkulationsmessung an (EDTNA-ERCA Journal 19, 6 (1993)).

Wenn von einer Bestimmung der Fistelrezirkulation und/oder des kardiopulmonalen Rezirkulationsanteils für zwei Blutflussraten die Rede ist, bedeutet dies nicht, dass die Fistelrezirkulation und/oder der kardiopulmonale Rezirkulationsanteil nicht auch aus der Rezirkulation für mehr als zwei Blutflussraten bestimmt werden könnte. Beispielsweise können mehrere aufeinander folgende Messungen vorgenommen und Mittelwerte gebildet werden.

Die erste Rezirkulationsmessung erfolgt bei einem hohen Blutfluss ($Q_{BH}$), bei der eine Fistelrezirkulation auftreten kann, während die zweite Messung bei einem niedrigen Blutfluss ($Q_{BL}$) erfolgt, bei dem eine Fistelrezirkulation nicht auftritt. Diese beiden Messwerte bilden die Grundlage für die Bestimmung der Fistelrezirkulation und/oder kardiopulmonalen Rezirkulation.

Die erfindungsgemäße Vorrichtung sieht die Berechnung der Fistelrezirkulation und/oder des kardiopulmonalen Rezirkulationsanteils auf der Grundlage einer Gleichung vor, die als Größen den Blutfluss ($Q_{BH}$) bei der ersten Messung und den Blutfluss ($Q_{BL}$) bei der zweiten Messung sowie die ermittelten Werte für die Rezirkulation bei der ersten und zweiten Messung ($R_H$ bzw. $R_L$) enthält.

Labormessungen haben gezeigt, dass Fistelrezirkulation und/oder des kardiopulmonalen Rezirkulationsanteils nach dem erfindungsgemäßen Verfahren mit hoher Genauigkeit berechnet werden können.

Die erfindungsgemäße Vorrichtung zum Bestimmen der Fistelrezirkulation und/oder des kardiopulmonalen Rezirkulationsanteils verfügt über eine Einrichtung zum Ändern einer physikalischen oder chemischen Kenngröße im Blut und eine Einrichtung zum Messen der Änderung der physikalischen oder chemischen Kenngröße im Blut. Die physikalische oder chemische Kenngröße kann beispielsweise die Konzentration eines Stoffes im Blut oder die Temperatur des Bluts sein. Vorzugsweise wird die Temperatur des Bluts verändert. Die Veränderung der Bluttemperatur erfolgt vorzugsweise durch Änderung der Temperatur der Dialysierflüssigkeit, wobei sich der Temperaturbolus von der Dialysierflüssigkeitsseite über den Dialysator auf die Blutseite fortpflanzt.

Darüber hinaus verfügt die erfindungsgemäße Vorrichtung über eine Rechen- und Auswerteeinheit, die derart ausgebildet ist, dass die Fistelrezirkulation und/oder der kardiopulmonale Rezirkulationsanteil auf der Grundlage der gemessenen physikalischen oder chemischen Kenngröße bei einer ersten und zweiten Blutflussrate bestimmbar ist.

Bei einer bevorzugten Ausführungsform der erfindungsgemäßen Vorrichtung ist die Einrichtung zum Ändern der physikalischen oder chemischen Kenngröße im Blut eine Einrichtung zur Veränderung der Temperatur des Bluts und die Einrichtung zum Messen der physikalischen oder chemischen Kenngröße im Blut eine Einrichtung zum Messen der Temperatur des Bluts.

Die erfindungsgemäße Vorrichtung zum Bestimmen der Fistelrezirkulation und/oder des kardiopulmonalen Rezirkulationsanteils kann eine separate Baugruppe bilden oder Bestandteil der Blutbehandlungsvorrichtung sein, die über einen extrakorporalen Blutkreislauf mit einem Dialysator verfügt, der durch eine semipermeable Membran in eine Blutkammer und eine Dialysierflüssigkeitskammer unterteilt ist.

Im Folgenden wird ein Ausführungsbeispiel der Erfindung unter Bezugnahme auf die Zeichnungen näher erläutert.

Es zeigen:

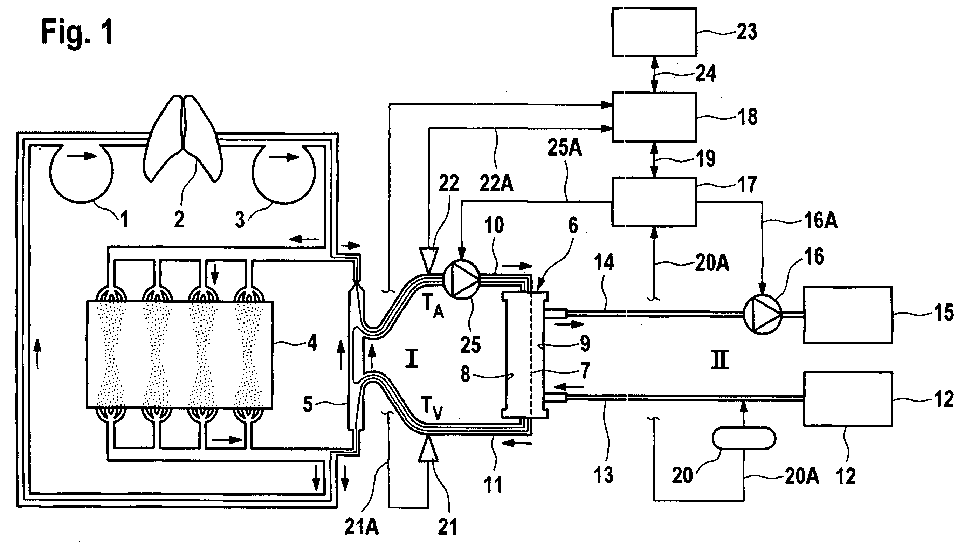

Fig. 1 die erfindungsgemäße Vorrichtung zur Bestimmung der Fistelrezirkulation und/oder des kardiopulmonalen Rezirkulationsanteils zusammen mit einer Blutbehandlungsvorrichtung einschließlich des intrakorporalen Blutkreislaufs in schematischer Darstellung,

Fig. 2 eine schematische Darstellung, die den extra- und intrakorporalen Blutkreislauf zeigt und

Fig. 3 den zeitlichen Verlauf von arterieller und venöser Fisteltemperatur und Dialysattemperatur während einer Rezirkulationsmessung.

Bei dem vorliegenden Ausführungsbeispiel ist die Vorrichtung zur Bestimmung der Fistelrezirkulation und/oder des kardiopulmonalen Rezirkulationsanteils Bestandteil der extrakorporalen Blutbehandlungsvorrichtung, wobei die Vorrichtung zur Bestimmung der Rezirkulationsanteile von einigen Komponenten der Dialysevorrichtung Gebrauch macht. Der intrakorporale Kreislauf umfasst das rechte Ventrikel 1 des Herzens, die Lunge 2, das linke Ventrikel 3 und sämtliche Kapillarsysteme 4 der inneren Organe, der Muskulatur und Haut etc. Um einen Zugang zu dem Blutgefäß zu schaffen, ist eine arteriovenöse Fistel 5 angelegt.

Die Dialysevorrichtung weist einen Dialysator 6 auf, der durch eine semipermeable Membran 7, in eine Blutkammer 8 und eine Dialysierflüssigkeitskammer 9 getrennt ist. Von dem arteriellen Teil der Fistel 5 geht über einen nicht dargestellten arteriellen Anschluss eine arterielle Blutleitung 10 ab, die zu einem Einlass der Blutkammer 8 führt, und von einem Auslass der Blutkammer 8 des Dialysators 6 geht eine venöse Blutleitung 11 ab, die über einen nicht dargestellten venösen Patientenanschluss zu dem venösen Teil der Fistel 5 führt. In der arteriellen Blutleitung 10 ist eine Blutpumpe 25 angeordnet, die im extrakorporalen Blutkreislauf I das Blut mit einer vorgegebenen Blutflussrate fördert.

Die Dialysierflüssigkeit wird von einer Einrichtung 12 bereitgestellt, von der eine Dialysierflüssigkeitszuführleitung 13 zu einem Einlass der Dialysierflüssigkeitskammer 9 führt, während von einem Auslass der Dialysierflüssigkeitskammer 9 des Dialysators 6 eine Dialysierflüssigkeitsabführleitung 14 des Dialysierflüssigkeitswegs II zu einem Auslass 15 führt. In die Dialysierflüssigkeitsabführleitung 14 ist eine Dialysierflüssigkeitspumpe 16 geschaltet.

Die Dialysevorrichtung verfügt über eine zentrale Steuereinheit (Mikroprozessor) 17, die über Steuerleitungen 25A und 16A mit der Blutpumpe 25 und der Dialysierflüssigkeitspumpe 16 verbunden ist. Die Steuereinheit 17 gibt die Förderraten der Pumpen 25, 16 vor, so dass sich im extrakorporalen Blutkreislauf I eine bestimmte Blutflussrate $Q_B$ und im Dialysierflüssigkeitsweg II eine bestimmte Dialysierflüssigkeitsrate $Q_D$ einstellt.

Die Vorrichtung zur Bestimmung der Fistelrezirkulation und/oder des kardiopulmonalen Rezirkulationsanteils weist eine Rechen- und Auswerteeinheit 18 auf, die über eine Datenleitung 19 mit der zentralen Steuereinheit 17 in Verbindung steht. Darüber hinaus weist die Vorrichtung eine Einrichtung 20 zum Ändern einer physikalischen oder chemischen Kenngröße im Blut auf. Die Einrichtung 20 ist bei dem vorliegenden Ausführungsbeispiel eine Einrichtung, mit der die Temperatur im Blut kurzzeitig verändert wird. Diese kurzzeitige Änderung der Temperatur im Blut wird als Temperaturbolus bezeichnet.

Bei dem vorliegenden Ausführungsbeispiel erzeugt die Einrichtung 20 einen Temperaturbolus im Dialysierflüssigkeitsweg II stromauf der Dialysierflüssigkeitskammer 9 des Dialysators 6. Zur Erzeugung des Temperaturbolus wird die Dialysattemperatur typischerweise um ca. 2,5 °C für 2,5 Minuten angehoben oder abgesenkt, um dann wieder auf den ursprünglichen Wert zurückgestellt zu werden. Die Einrichtung 20 zur Erzeugung des Temperaturbolus ist über eine Steuerleitung 20A mit der Steuereinheit 17 der Dialysevorrichtung verbunden.

Zum Nachweis der Änderung der physikalischen oder chemischen Kenngröße auf der Blutseite dient eine Einrichtung, die einen nicht-invasiven venösen Temperatursensor 21 zum Messen der venösen Bluttemperatur $T_V$ und einen nicht-invasiven arteriellen Temperatursensor 22 zum Messen der arteriellen Bluttemperatur $T_A$ umfasst. Die venösen und arteriellen Temperatursensoren 21, 22 sind über Datenleitungen 21A und 22A mit der Rechen- und Auswerteeinheit 18 verbunden.

Nachfolgend wird die Funktionsweise der Dialysevorrichtung im Einzelnen erläutert.

Das vom linken Ventrikel 1 emittierte Blut fließt zum größten Teil in die Kapillarsysteme aller Organe und zu einem kleinen Teil in die Fistel 5. Für den Fall, dass der Blutfluss im extrakorporalen Kreislauf kleiner als der Fluss des in die Fistel bzw. aus der Fistel fließenden Bluts ist, fließt das Fistelblut zum einen Teil durch den extrakorporalen Blutkreislauf II und zum anderen Teil durch die Fistel 5. Das extrakorporale Blut, das durch die Fistel fließende Blut und das aus den Kapillarsystemen stammende Blut vereinigen sich schließlich wieder im Rücklauf zum Herzen. Wenn der extrakorporale Blutfluss hingegen größer als der Fistelfluss ist, rezirkuliert Blut aus dem extrakorporalen Blutkreislauf, wobei die Fistel vom venösen zum arteriellen Anschluss durchflossen wird.

Zur Bestimmung der Fistelrezirkulation und des kardiopulmonalen Rezirkulationsanteils wird zunächst die Summe von Fistelrezirkulation und kardiopulmonalem Rezirkulationsanteil ermittelt, die als Rezirkulation R bezeichnet wird. Zunächst steuert die Steuereinheit 17 die Einrichtung 20 zur Erzeugung des Temperaturbolus im Dialysierflüssigkeitsweg II stromauf der Dialysierflüssigkeitskammer 9 des Dialysators 6 an, so dass die Temperatur der Dialysierflüssigkeit typischerweise um ca. 2,5 °C für 2,5 Minuten verändert, beispielsweise erhöht wird. Danach wird wieder der zu Beginn des Bolus geltende Sollwert für die Dialysattemperatur eingestellt.

Der Temperaturbolus wird über den Dialysator 6 in den extrakorporalen Blutkreislauf I übertragen und führt im Blutkreislauf I zu einer Erhöhung oder Reduzierung der Temperatur des von dem Dialysator 6 zu dem Patienten strömenden venösen Bluts. Diese Temperaturänderung erfasst der venöse Temperatursensor 21. Der sich aufgrund der Fistelrezirkulation und der kardiopulmonalen Rezirkulation fortpflanzende venöse Temperaturbolus wird als abgeschwächter Temperaturbolus mit dem arteriellen Temperatursensor 22 erfasst. Die Rechen- und Auswerteeinheit 18 empfängt die Messwerte des venösen und arteriellen Temperatursensors 21, 22 und speichert die Messwerte. Des Weiteren empfängt die Rechen- und Auswerteeinheit 18 den Wert der Blutflussrate $Q_B$, die von der Steuereinheit 17 vorgegeben wird. Aus den beiden Messwerten für die arterielle und venöse Temperatur $T_A$ und Tv berechnet die Rechen- und Auswerteeinheit 18 die Rezirkulation.

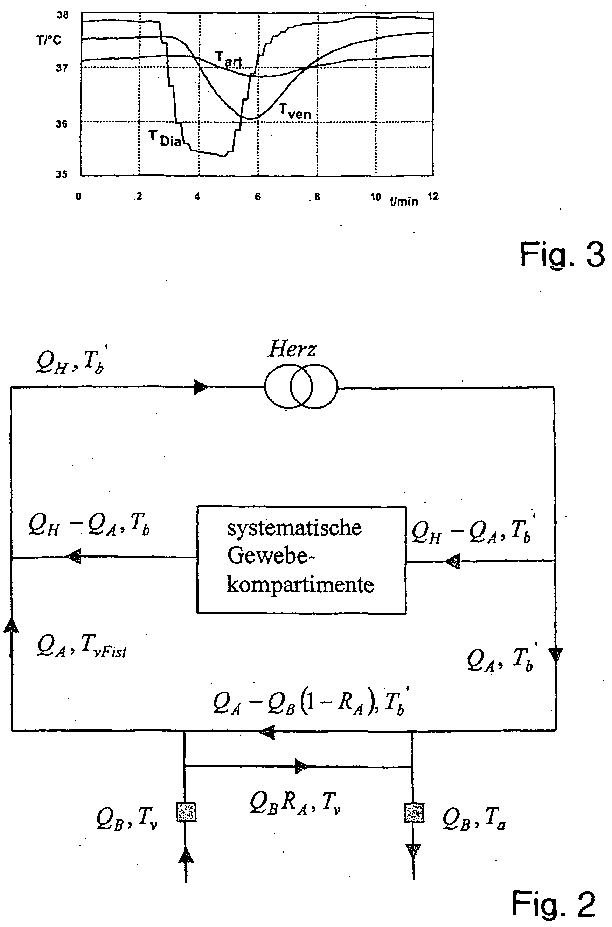

Fig. 3 zeigt die Temperatur der Dialysierflüssigkeit $T_{DIA}$ stromauf des Dialysators 6 sowie die Temperatur des venösen und arteriellen Bluts $T_V$ und $T_A$ als Funktion der Zeit. Das Verhältnis der Bolusgröße des arteriellen Antwortbolus $T_A$ zum venösen Stimulusbolus $T_V$ entspricht der Summe von Fistelrezirkulation und kardiopulmonalem Rezirkulationsanteil. Die Rechen- und Auswerteeinheit 18 ermittelt die Größen des arteriellen und venösen Temperaturbolus durch Integration oder über die Amplituden und berechnet aus dem Verhältnis der beiden Temperatur-Integrale nach den im Stand bekannten Verfahren die Rezirkulation. Da die Bestimmung der Rezirkulation zum Stand der Technik gehört, wird darauf verzichtet, das Verfahren im Einzelnen zu beschreiben. Als Offenbarung wird beispielsweise auf das in der Zeitschrift EDTNA-ERCA Journal 19, 6 (1993) beschriebene Verfahren nach dem Prinzip der Thermodilution verwiesen. Dieses Verfahren zur Messung der Gesamt-Rezirkulation, die sich aus Fistel- und kardiopulomaler Rezirkulation zusammensetzt, ist auch in DRUKKER, PARSONS and MAHER, Replacement of Renal Function by Dialysis, 5th Edition, 2004, Kluwer Academic Publishers BV beschrieben. Darüber hinaus ist es auch möglich die Gesamtrezirkulation für die beiden Blutflüsse nach dem Verfahren zu bestimmen, das in der DE 197 02 441 C1 beschrieben ist.

Die Erfindung beruht darauf, die Rezirkulation nach den bekannten Verfahren in einer ersten Messung bei einer ersten Blutflussrate $Q_{BH}$ und in einer darauf folgenden zweiten Messung für eine zweite Blutflussrate $Q_{BL}$ zu bestimmen, wobei die beiden Blutflussraten sich voneinander unterscheiden. Eine der beiden Blutflussraten, beispielsweise die erste Blutflussrate $Q_{BH}$ ist derart bemessen, dass eine Fistelrezirkulation $R_A$ auftritt, während die andere Blutflussrate $Q_{BL}$ derart bemessen ist, dass eine Fistelrezirkulation nicht auftritt.

Eine Fistelrezirkulation tritt dann auf, wenn der Blutfluss größer als der Fistelfluss ist, d.h. mehr Blut aus der Fistel abgezogen wird, als der Fistel zufließt. Demgegenüber tritt eine Fistelrezirkulation nicht auf, wenn der Fistelfluss deutlich größer als der Blutfluss ist. Als Werte für den höheren Blutfluss, bei der eine Fistelrezirkulation auftritt, und den niedrigeren Blutfluss, bei dem keine Fistelrezirkulation auftritt, können Erfahrungswerte herangezogen werden. Dabei ist davon auszugehen, dass der Fistelfluss bei den in der Praxis vorgegebenen Bedingungen zwischen 800 und 1.000 ml/min., im Allgemeinen aber nicht über 1.500 ml/min. liegt.

Die Rechen- und Auswerteeinheit 18 berechnet aus den beiden Werten für die Rezirkulation $R_H$, $R_L$ bei der höheren und niedrigeren Blutflussrate $Q_{BH}$, $Q_{BL}$, bei der eine Fistelrezirkulation auftritt bzw. nicht auftritt, die Fistelrezirkulation $R_A$, die von der Blutflussrate abhängig ist. Dann berechnet die Rechen- und Auswerteeinheit 18 aus der für eine vorgegebene Blutflussrate ermittelten Rezirkulation R und der berechneten Fistelrezirkulation $R_A$ den kardiopulmonalen Rezirkulationsanteil $R_{CP}$.

Die Berechnung der Fistelrezirkulation $R_A$ und des kardiopulmonalen Rezirkulationsanteils $R_{CP}$ erfolgt mit einem im Rahmen der in der Praxis erforderlichen Genauigkeit hinreichenden Näherungsverfahren, das nachfolgend im Einzelnen beschrieben wird.

Fig. 2 zeigt in vereinfachter schematischer Darstellung die Flüsse und Temperaturen im intra- und extrakorporalen Kreislauf, wobei

| | |
|---|---|
| $Q_H$: | Herzminutenvolumen |
| $Q_A$: | Fistelfluss |
| $Q_B$: | Blutfluss |
| $Q_{RA}$: | Anteil des Rezirkulationsflusses in der Fistel |
| $R_A$: | Rezirkulation in der Fistel |
| $R_{CP}$ | kardiopulmonale Rezirkulation (Fistelanteil am Herzminutenvolumen) |
| $R_b$ | Blutflussanteil am Herzminutenvolumen |
| $R_{CP}$: | kardiopulmonaler Rezirkulationsanteil |
| $R$: | gesamte Rezirkulation im extrakorporalen Blutkreislauf |
| $R_H$: | gemessene Rezirkulation bei einem hohen Blutfluss $Q_{BH}$ |
| $R_L$: | gemessene Rezirkulation bei einem niedrigen Blutfluss $Q_{BL}$ |
| $T_v$: | gemessene venöse Fisteltemperatur |
| $Ta$: | gemessene arterielle Fisteltemperatur |
| $T_{vFistel}$: | venöse Fisteltemperatur |
| $T_b$: | Körpertemperatur |
| $T_b'$: | Körpertemperatur |
| $Q_{UF}$: | Ultrafiltrationsrate |

ist.

Wenn eine Rezirkulation in der Fistel auftritt, setzt sich die gesamte Rezirkulation R aus dem Fistel- und dem kardiopulmonalen Rezirkulationsanteil zusammen:

Die Gesamtrezirkulation R gibt den Anteil des bereits gereinigten extrakorporalen Blutflusses $Q_{bereits\ gereinit}$, der über den arteriellen Zugang wieder in den extrakorporalen Blutkreislauf eintritt, ohne dass zuvor im (großen) Körperkreislauf ein Konzentrationsausgleich stattgefunden hat, an dem Blutfluss $Q_B$ an $Q_{bereits\ gereinigt}$ / $Q_B$).

Die Gesamtrezirkulation R setzt sich aus einem Anteil des Flusses des gereinigten Blutes $R_{CP}$, der direkt nach dem Lungenkreislauf wieder in den extrakorporalen Kreislauf übergeht, ohne dass es zuvor zu einem Konzentrationsausgleich im großen Körperkreislauf gekommen ist, und der Fistelrezirkulation $R_A$ zusammen.

$$R = R_{CP} + R_A \qquad (1)$$

Der Anteil des Flusses des gereinigten Blutes $R_{CP}$ wird in der Literatur auch als kardiopulmonale Rezirkulation bezeichnet. In der Literatur wird mit einer kardiopulmonalen Rezirkulation aber auch der Anteil des Herzminutenvolumens ($Q_H$) am Blutfluss durch den arterio-venösen Shunt ($Q_A$)) bezeichnet ($R_{CP} = Q_A / Q_H$). Zur Abgrenzung von diesem Blutfluss wird der Anteil des Flusses des gereinigten Blutes, der direkt nach dem Lungenkreislauf wieder in den extrakorporalen Kreislauf ohne einen Konzentrationsausgleich übergeht, hier als kardiopulmonaler Rezirkulationsanteil, d.h. als Anteil der kardiopulmonalen Rezirkulation an der Gesamtrezirkulation $R_{CP}$ bezeichnet. Dieser berechnet sich nach Schneditz mit $R_{CP} = Q_B / Q_H - Q_A + Q_B$) (Schneditz et al. Seminars in Dialysis - Vol. 16 Nr.6, 2003, pp.483-487).

Die Fistelrezirkulation $R_A$ ist als der Anteil des dialysierten und mit der venösen Blutleitung zur Fistel zurückgeführten Blutflusses an dem extrakorporalen Blutfluss $Q_B$ definiert, der über eine (partielle) Stromumkehr im Shunt wieder über die arterielle Blutleitung dem Dialysator zugeführt wird ($R_A = Q_{Flussumkehr} / Q_B$).

Das als Thermodilution bekannte Verfahren zum Messen der Rezirkulation berücksichtigt alle Flüsse und Temperaturen, die in Fig. 2 angegeben sind, wobei angenommen wird, dass ein Austausch mit der Umgebung nicht stattfindet. $T_b$ ist die Körpertemperatur, die sich aus der Mikrozirkulation in den systematischen Gewebekompartimenten ergibt. $T_{vFist}$ ist die Fisteltemperatur. Nach der Mischung mit dem Fistelfluss ergibt sich eine Körpermischtemperatur $T_{b'}$, mit der das Blut zum Herz und weiter zur Fistel gelangt. In der Fistel finden weitere Temperaturmischvorgänge statt.

Nach dem Prinzip der Wärmebilanz gilt:

$$Q_H T_b^{'} = \left(Q_H - Q_A\right) T_b + Q_A T_{vFist}$$

$$Q_A T_{vFist} = \left(Q_A - Q_B\left(1 - \bar{R}_A\right)\right) T_b^{'} + Q_B\left(1 - \bar{R}_A\right) T_v$$

mit $R_{cp} = Q_A / Q_H$ Fistelflussanteil am Herzminutenvolumen (= kardiopulmonale Rezirkulation)
*und* $R_b = Q_B / Q_H$ Blutflussanteil am Herzminutenvolumen

$$T_b^{'} = \frac{T_a - R_A T_v}{1 - R_A}$$

$$T_b = \frac{\left(1 - R_{cp} + R_b\left(1 - R_A\right)\right) T_b^{'} - R_b\left(1 - R_A\right) T_v}{1 - R_{cp}}$$

eingesetzt und in folgende Form gebracht $T_b = \dfrac{T_a - R T_v}{1 - R}$

$$R = \frac{R_b\left(1 - R_A\right) + R_A\left(1 - R_{cp}\right)}{1 - R_{cp} + R_b\left(1 - R_A\right)}$$

Unter Berücksichtigung des Ultrafiltrationseinflusses ergibt sich aus einer ähnlichen Herleitung nach dem Prinzip der Wärmebilanz folgender Zusammenhang:

$$R = \frac{(R_b - R_{uf})(1 - R_A) + R_A(1 - \frac{Q_{uf}}{Q_B})(1 - R_{cp} + R_{uf})}{1 - R_{cp} + R_b(1 - R_A) + R_A R_{uf}}$$

wobei $Q_{uf}$ die Ultrafiltrationsrate (ml/min) und

$$R_{uf} = Q_{uf} / Q_H \text{ ist.}$$

In der Praxis ist der Einfluss der Ultrafiltration vernachlässigbar. Damit wird die obige Gleichung

$$\text{zu } R = \frac{R_b\left(1 - R_A\right) + R_A\left(1 - R_{cp}\right)}{1 - R_{cp} + R_b\left(1 - R_A\right)} \text{ , wenn } Q_{uf} = 0."$$

In dem Fall, dass eine Fistelrezirkulation nicht auftritt ($R_A = 0$) :

$$R = \frac{R_b}{1 - R_{cp} + R_b} = \frac{Q_B}{Q_H - Q_A + Q_B} \tag{2}$$

Nach einer Umformung von $R = \dfrac{R_b\left(1 - R_A\right) + R_A\left(1 - R_{cp}\right)}{1 - R_{cp} + R_b\left(1 - R_A\right)}$ ergibt sich

$$R = \frac{Q_B\left(1 - R_A\right) + R_A\left(Q_H - Q_A\right)}{Q_H - Q_A + Q_B\left(1 - R_A\right)} \tag{3}$$

Aus der 1. Rezirkulations-Messung bei dem niedrigen Blutfluss von $Q_{BL}$ unter der Annahme von $R_A = 0$ :

$$R_L = \frac{Q_{BL}}{Q_H - Q_A + Q_{BL}}$$

$$Q_H - Q_A = \frac{1 - R_L}{R_L} \cdot Q_{BL} \tag{4}$$

Aus der 2. Rezirkulations-Messung bei dem hohen Blutfluss von $Q_{BH}$ unter der Annahme von $RA \neq 0$ :

$$R_H = \frac{Q_{BH}\left(1 - R_A\right) + R_A\left(Q_H - Q_A\right)}{Q_H - Q_A + Q_{BH}\left(1 - R_A\right)} \tag{5}$$

Aus (4) und (5),

$$R_A = \frac{\left(1 - R_L\right)R_H k - R_L\left(1 - R_H\right)}{\left(1 - R_L\right)k - R_L\left(1 - R_H\right)} \tag{6}$$

wobei $k = \dfrac{Q_{BL}}{Q_{BH}}$ .

Die Fistelrezirkulation $R_A$ berechnet die Rechen- und Auswerteeinheit 18 nach der ersten und zweiten Rezirkulationsmessung zur Bestimmung der Rezirkulation unter der Annahme $R_A$ gleich Null bzw. unter der Annahme $R_A$ ungleich Null aus den ermittelten Werten für $R_L$ und $R_H$ sowie dem niedrigen Blutfluss $Q_{BL}$ für $R_A$ gleich Null und dem hohen Blutfluss $Q_{BH}$ für $R_A$ ungleich Null nach Gleichung (6).

Anschließend berechnet die Rechen- und Auswerteeinheit 18 nach Gleichung (1) den kardiopulmonalen Rezirkulationsanteil $R_{CP}$ in Abhängigkeit vom Blutfluss $Q_B$. Hierfür bildet die Rechen- und Auswerteeinheit die Differenz zwischen der für einen bestimmten Blutfluss $Q_B$ gemessenen Rezirkulation R, beispielsweise die in der vorausgehenden ersten Rezirkulationsmessung gemessenen Rezirkulation, und der zuvor berechneten Fistelrezirkulation $R_A$.

Die beiden Werte für die Fistelrezirkulation $R_A$ und den kardiopulmonalen Rezirkulationsanteil $R_{CP}$ werden auf einer Anzeigeeinheit 23, beispielsweise einem Display, angezeigt, die über eine Datenleitung 24 mit der Rechen- und Auswerteeinheit 18 verbunden ist.

Aus den ermittelten Werten für die Fistelrezirkulation $R_A$ und den kardiopulmonalen Rezirkulationsanteil $R_{CP}$

können in der Rechen- und Auswerteeinheit nach den im Stand der Technik bekannten Gleichungen noch weitere Größen berechnet werden, die für die Dialysebehandlung relevant sind.

Eine alternative Ausführungsform sieht die nachfolgend beschriebene Auswertung der Messergebnisse vor.

Der kardiopulmonale Rezirkulationsanteil $R_{CP}$ berechnet sich nach der Schneditz-Formel (Schneditz D, Kaufman AM, Levin N: Surveillance of access function by the blood temperature monitor, Semin Dial 16(6) (2003) 483-7) unter der Annahme, dass eine Rezirkulation in der Fistel nicht auftritt, nach der folgenden Gleichung:

$$R_{CP} = \frac{Q_B}{Q_H - Q_A + Q_B} \qquad (7)$$

Wenn eine Fistelrezirkulation auftritt, berechnet sich die gesamte Rezirkulation aus der Summe der Fistelrezirkulation $R_A$ und dem kardiopulmonalen Rezirkulationsanteil $R_{CP}$:

$$R = R_A + R_{CP} \qquad (8)$$

Die erste Rez.-Messung bei einem hohen Blutfluss $Q_{BH}$ (möglicher Auftritt der Fistelrezirkulation):

$$R_H = \frac{Q_{RA}}{Q_{BH}} + \frac{Q_{BH} - Q_{RA}}{Q_H - Q_A + (Q_{BH} - Q_{RA})} = \frac{Q_{RA}}{Q_{BH}} + \frac{1 - Q_{RA}/Q_{BH}}{(Q_H - Q_A)/Q_{BH} + (1 - Q_{RA}/Q_{BH})}$$

$$R_H = R_A + \frac{1 - R_A}{(Q_H - Q_A)/Q_{BH} + (1 - R_A)} \qquad (9)$$

Die zweite Rez.-Messung bei einem niedrigen Blutfluss $Q_{BL}$ (kein Auftritt von Fistelrezirkulation):

$$R_L = \frac{Q_{BL}}{Q_H - Q_A + Q_{BL}}$$

$$Q_H - Q_A = Q_{BL} \cdot (\frac{1}{R_L} - 1) \qquad (10)$$

Aus (9) uns (10):

$$R_A^2 - (R_H + k) \cdot R_A + R_H(1 + k) - 1 = 0 \qquad (11)$$

wobei

$$k = \frac{Q_{BL}}{Q_{BH}} \cdot (\frac{1}{R_L} - 1)$$

Die sinnvolle Lösung der G1. (11) ist

$$R_A = 0,5 \left[ R_H + k - \sqrt{(k - R_H)^2 + 4(1 - R_H)} \right] \qquad (12)$$

Bei der alternativen Ausführungsform berechnet die Rechen- und Auswerteeinheit 18 nach Durchführung der beiden Rezirkulationsmessungen zur Bestimmung der Rezirkulationen $R_H$ und $R_L$ bei hohem und niedrigem Blutfluss $Q_{BH}$ und $Q_{BL}$, bei denen eine Fistelrezirkulation auftritt bzw. nicht auftritt, die Fistelrezirkulation $R_A$ nach Gleichung (12).

Anschließend berechnet die Rechen- und Auswerteeinheit 18 für einen bestimmten Blutfluss $Q_B$ den kardiopulmonalen Rezirkulationsanteil $R_{CP}$ aus der für diesen Blutfluss bestimmten Rezirkulation R und der berechneten Fistelrezirkulation $R_A$ nach Gleichung (8).

Auf die zweite Messung einer physikalischen oder chemischen Kenngröße im Blut bei einer niedrigeren Blutflussrate kann grundsätzlich verzichtet werden, wenn aufgrund der ersten Messung, die bei einer höheren Butflussrate erfolgt, das Auftreten einer Fistelrezirkulation als unwahrscheinlich abgeschätzt werden kann. Eine alternative Ausführungsform sieht daher periodische Messungen einer physikalischen oder chemischen Kenngröße im Blut bei einem höheren Blutfluss $Q_{BH}$ zur Ermittlung der Summe von Fistelrezirkulation und kardiopulmonalem Rezirkulationsanteil, d.h. der Gesamtrezirkulation vor, wobei eine zweite Messung der physikalischen oder chemischen Kenngröße bei einem niedrigeren Blutfluss nur dann erfolgt, wenn bei der ersten Messung ein vorgegebener Grenzwert überschritten wird. Der vorgegebene Grenzwert kann ein Wert sein, der auf Erfahrungswerten beruht. Als Grenzwert kann die Untergrenze des kardiopulmonalen Rezirkulationsanteils $R_{CP}$ herangezogen werden, der beispielsweise 5 - 10 %, insbesondere 6 - 8 % beträgt. Es können aber auch andere Erfahrungswerte herangezogen werden.

## Patentansprüche

1. Vorrichtung zum Bestimmen der Rezirkulation ($R_A$) in einer Fistel und/oder des kardiopulmonalen Rezirkulationsanteils ($R_{CP}$) für eine Blutbehandlungsvorrichtung, bei der das zu behandelnde Blut in einem extrakorporalen Blutkreislauf durch die Blutkammer eines durch eine semipermeable Membran in die Blutkammer und eine Dialysierflüssigkeitskammer unterteilten Dialysators strömt und Dialysierflüssigkeit in einem Dialysierflüssigkeitsweg die Dialysierflüssigkeitskammer des Dialysators durchströmt, wobei in dem extrakorporalen Kreislauf eine Blutpumpe angeordnet ist, mit
einer Einrichtung (20) zum Ändern einer physikalischen oder chemischen Kenngröße im Blut,
einer Einrichtung (21, 22) zum Messen der Veränderung der physikalischen oder chemischen Kenngröße bei einer ersten Blutflussrate ($Q_{BH}$) im Blut, die auf eine erste Änderung der physikalischen oder chemischen Größe zurückzuführen ist, und zum Messen der Veränderung der physikalischen oder chemischen Kenngröße bei einer zweiten Blutflussrate ($Q_{BL}$) im Blut, die auf eine zweite Änderung der physikalischen oder chemischen Größe zurückzuführen ist, wobei die erste Butflussrate ($Q_{BH}$) größer als die zweite Blutflussrate ($Q_{BL}$) ist und die erste Butflussrate ($Q_{BH}$) derart bemessen ist, dass eine Fistelrezirkulation auftritt und die zweite Butflussrate ($Q_{BH}$) derart bemessen ist, dass eine Fistelrezirkulation nicht auftritt,
einer Rechen- und Auswerteeinheit (18), die derart ausgebildet ist, dass die Fistelrezirkulation ($R_A$) und/oder der kardiopulmonale Rezirkulationsanteil ($R_{CP}$) auf der Grundlage der gemessenen physikalischen oder chemischen Kenngröße bei der ersten und zweiten Blutflussrate ($Q_{SH}$, $Q_{BL}$) bestimmbar ist.

2. Vorrichtung nach Anspruch 1, **dadurch gekennzeichnet, dass** die Rechen- und Auswerteeinheit (18) derart ausgebildet ist, dass auf der Grundlage der gemessenen physikalischen oder chemischen Kenngröße bei der ersten Blutflussrate ($Q_{BH}$) die Summe ($R_H$) von der Fistelrezirkulation ($R_A$) und dem kardiopulmonalen Rezirkulationsanteil ($R_{CP}$) für die erste Blutflussrate, und auf der Grundlage der gemessenen physikalischen oder chemischen Kenngröße bei der zweiten Blutflussrate ($Q_{BL}$ die Summe ($R_L$ von Fistelrezirkulation ($R_A$) und kardiopulmonalem Rezirkulationsanteil ($R_{CP}$) für die zweite Blutflussrate bestimmbar sind.

3. Vorrichtung nach Anspruch 1 oder 2, **dadurch gekennzeichnet, dass** die Rechen- und Auswerteeinheit (18) derart ausgebildet ist, dass die Fistelrezirkulation ($R_A$) aus den bei der ersten und zweiten Blutflussrate ($Q_{BH}$ und $O_{BL}$) ermittelten Summen ($R_H$ und $R_L$) von dem kardiopulmonalen Rezirkulationsanteil ($R_{CP}$) und der Fistelrezirkulation ($R_A$) nach der folgenden Gleichung bestimmbar ist:

$$R_A = \frac{(1-R_L)R_H k - R_L(1-R_H)}{(1-R_L)k - R_L(1-R_H)}$$

wobei $k = \dfrac{Q_{BL}}{Q_{BH}}$

4. Vorrichtung nach Anspruch 1 oder 2, **dadurch gekennzeichnet, dass** die Rechen- und Auswerteeinheit (18) derart ausgebildet ist, dass die Fistelrezirkulation ($R_A$) aus den bei der ersten und zweiten Blutflussrate ($Q_{BH}$ und $Q_{BL}$) ermittelten Summen ($R_H$ und $R_L$) von dem kardiopulmonalen Rezirkulationsanteil ($R_{CP}$) und der Fistelrezirkulation

($R_A$) nach der folgenden Gleichung bestimmbar ist

$$k = \frac{Q_{BL}}{Q_{BH}} \cdot \left(\frac{1}{R_L} - 1\right)$$

$$R_A = 0{,}5\left[ R_H + k - \sqrt{(k - R_H)^2 + 4(1 - R_H)} \right].$$

5. Vorrichtung nach Anspruch 3 oder 4, **dadurch gekennzeichnet, dass** die Rechen- und Auswerteeinheit (18) Mittel zum Bilden der Differenz von der Summe von dem kardiopulmonalen Rezirkulationsanteil ($R_{CP}$) und der Fistelrezirkulation ($R_A$) einerseits und der Fistelrezirkulation ($R_A$) andererseits zur Bestimmung des kardiopulmonalen Rezirkulationsanteils ($R_{CP}$) aufweist.

6. Vorrichtung nach einem der Ansprüche 1 bis 5, **dadurch gekennzeichnet, dass** die Einrichtung (20) zum Ändern einer physikalischen oder chemischen Kenngröße im Blut eine Einrichtung zur Veränderung der Temperatur (T) des Bluts ist.

7. Vorrichtung nach einem der Ansprüche 1 bis 6, **dadurch gekennzeichnet, dass** die Einrichtung (21, 22) zum Messen einer physikalischen oder chemischen Kenngröße im Blut eine Einrichtung zum Messen der Temperatur des Bluts ist.

8. Blutbehandlungsvorrichtung mit einem durch eine semipermeable Membran (7) in eine Blutkammer (8) und eine Dialysierflüssigkeitskammer (9) unterteilten Dialysators (6), einer zu der Blutkammer des Dialysators führenden arteriellen Blutleitung (10) und einer von der Blutkammer abgehenden venösen Blutleitung (11) eines extrakorporalen Blutkreislaufs (I), wobei in dem extrakorporalen Kreislauf eine Blutpumpe (25) angeordnet ist, einer zu der Dialysierflüssigkeitskammer (9) führtenden Dialysierflüssigkeitszuführleitung (13) und einer von der Dialysierflüssigiteitskammer (9) des Dialysators (6) abgehenden Dialysierflüssigkeitsabführleitung (14), **gekennzeichnet durch** eine Vorrichtung zum Bestimmen der Rezirkulation ($R_A$) in einer Fistel und/oder des kardiopulmonalen Rezirkulationsanteils ($R_{CP}$) nach einem der Ansprüche 1 bis 7.

**Claims**

1. Arrangement for determining recirculation ($R_A$) in a fistula and/or the cardiopulmonary proportion of recirculation ($R_{CP}$), for an extra-corporeal treatment of blood in which the blood to be treated flows in an extra-corporeal blood circuit through the blood chamber of a dialyser which is divided into the blood chamber and a dialysis-fluid chamber by a semi-permeable membrane and dialysis fluid flows through the dialysis-fluid chamber of the dialyser on a path for dialysis fluid, a blood pump being arranged in the extra-corporeal blood circuit, having
a means (20) of changing a physical or chemical parameter in the blood,
a means (21, 22) of measuring the change in the physical or chemical parameter at a first blood flow rate ($Q_{BH}$), in the blood, which change is attributable to the first change in the physical or chemical parameter, and of measuring the change in the physical or chemical parameter at a second blood flow rate ($Q_{BL}$), in the blood, which change is attributable to the second change in the physical or chemical parameter, the first blood rate ($Q_{BH}$) being greater than the second blood rate ($Q_{BL}$) and the first blood rate ($Q_{BH}$) being such that fistular recirculation occurs and the second blood rate ($Q_{BH}$) being such that fistular recirculation does not occur,
a computing and analysing unit (18) which is so designed that fistular recirculation ($R_A$) and/or the cardio-pulmonary proportion of recirculation ($R_{CP}$) can be determined on the basis of the physical or chemical parameter which is measured at the first and second blood flow rates ($Q_{BH}$, $Q_{BL}$).

2. Arrangement according to claim 1, **characterised in that** the computing and analysing unit (18) is so designed that on the basis of the physical or chemical parameter which is measured at the first blood flow rate ($Q_{BH}$), the sum ($R_H$) of fistular recirculation and the cardio-pulmonary proportion of recirculation can be determined for the first blood flow rate, and on the basis of the physical or chemical parameter which is measured at the second blood flow rate ($Q_{BL}$), the sum ($R_L$) of fistular recirculation and the cardio-pulmonary proportion of recirculation can be determined for the second blood flow rate.

3. Arrangement according to claim 1 or 2, **characterised in that** the computing and analysing unit (18) is so designed that fistular recirculation ($R_A$) can be determined from the sums ($R_H$ and $R_L$), as determined at the first and second blood flow rates ($Q_{BH}$ and $Q_{BL}$), of the cardio-pulmonary proportion of recirculation ($R_{CP}$) and fistular recirculation ($R_A$), from the following equation:

$$R_A = \frac{(1 - R_L)R_H k - R_L(1 - R_H)}{(1 - R_L)k - R_L(1 - R_H)}$$

where $k = \dfrac{Q_{BL}}{Q_{BH}}$ .

4. Arrangement according to claim 1 or 2, **characterised in that** the computing and analysing unit (18) is so designed that fistular recirculation ($R_A$) can be determined from the sums ($R_H$ and $R_L$), as determined at the first and second blood flow rates ($Q_{BH}$ and $Q_{BL}$), of the cardio-pulmonary proportion of recirculation ($R_{CP}$) and fistular recirculation ($R_A$), from the following equation:

$$k = \frac{Q_{BL}}{Q_{BH}} \cdot (\frac{1}{R_L} - 1)$$

$$R_A = 0.5 \left[ R_H + k - \sqrt{(k - R_H)^2 + 4(1 - R_H)} \right].$$

5. Arrangement according to claim 3 or 4, **characterised in that** the computing and analysing unit (18) has means for forming the difference between on the one hand the sum of the cardio-pulmonary proportion of recirculation ($R_{CP}$) and fistular recirculation ($R_A$), and on the other hand fistular recirculation ($R_A$).

6. Arrangement according to one of claims 1 to 5, **characterised in that** the means (20) of changing a physical or chemical parameter in the blood is a means of changing the temperature (T) of the blood.

7. Arrangement according to one of claims 1 to 6, **characterised in that** the means (21, 22) of measuring a change in a physical or chemical parameter in the blood is a means of measuring the temperature of the blood.

8. Blood-treating apparatus having a dialyser (6) which is divided into a blood chamber (8) and a dialysis-fluid chamber (9) by a semi-permeable membrane (7), having, as part of an extra-corporeal blood circuit (I), an arterial blood line (10) which leads to the blood chamber of the dialyser and a venous blood line (11) which runs from the blood chamber (8), a blood pump (25) being arranged in the extra-corporeal circuit, having a dialysis-fluid infeed line (13) which leads to the dialysis-fluid chamber (9) and a dialysis-fluid takeaway line (14) outgoing from the dialysis-fluid chamber (9) of the dialyser (6), **characterised by** an arrangement for determining fistular recirculation ($R_A$) and/or the cardio-pulmonary proportion of recirculation ($R_{CP}$) according to one of claims 1 to 7.

**Revendications**

1. Dispositif servant à déterminer la recirculation ($R_A$) dans une fistule et/ou la partie de recirculation cardio-pulmonaire ($R_{Cp}$) pour un dispositif de traitement du sang, dans lequel le sang à traiter circule dans un circuit de sang extracorporel à travers le compartiment de sang d'un dialyseur séparé par une membrane semi-perméable en le compartiment de sang et en un compartiment de liquide de dialyse et dans lequel le liquide de dialyse traverse, sur un trajet de liquide de dialyse, le compartiment de liquide de dialyse du dialyseur, une pompe à sang étant disposée dans le circuit extracorporel, comprenant un système (20) servant à modifier une caractéristique physique ou chimique dans le sang, un système (21, 22) servant à mesurer la variation de la grandeur caractéristique physique ou chimique dans le cas d'un premier débit sanguin ($Q_{BH}$) dans le sang, qui est à relier à une première modification de la grandeur physique ou chimique, et servant à mesurer la variation de la grandeur caractéristique physique ou chimique dans le cas d'un deuxième débit sanguin ($Q_{BL}$) dans le sang, qui est à relier à une deuxième modification de la grandeur physique ou chimique, le premier débit sanguin ($Q_{BH}$) étant plus grand que le deuxième débit sanguin ($Q_{BL}$) et le premier débit sanguin ($Q_{BH}$) étant ajusté de telle manière qu'une recirculation dans une fistule survient et le deuxième débit sanguin ($Q_{BL}$) étant ajusté de telle manière qu'aucune recirculation dans une fistule ne survient, une unité de calcul et d'analyse (18) qui est réalisée de telle manière que la recirculation dans une fistule ($R_A$) et/ou la partie de recirculation cardio-pulmonaire ($R_{CP}$) peuvent être déterminées sur la base de la grandeur caractéristique physique ou chimique mesurée dans le cas du premier et du deuxième débit sanguin ($Q_{BH}$, $Q_{BL}$).

2. Dispositif selon la revendication 1, **caractérisé en ce que** l'unité de calcul et d'analyse (18) est réalisée de telle manière que la somme ($R_H$) de la recirculation dans une fistule ($R_A$) et de la partie de recirculation cardio-pulmonaire ($R_{CP}$) peut être déterminée pour le premier débit sanguin sur la base de la grandeur caractéristique physique ou chimique mesurée dans le cas du premier débit sanguin ($Q_{BH}$) et que la somme ($R_L$) de la recirculation dans une fistule ($R_A$) et de la partie de recirculation cardio-pulmonaire ($R_{CP}$) pour le deuxième débit sanguin peut être déterminée sur la base de la grandeur caractéristique physique ou chimique mesurée dans le cas du deuxième débit sanguin ($Q_{BL}$).

3. Dispositif selon la revendication 1 ou 2, **caractérisé en ce que** l'unité de calcul et d'analyse (18) est réalisée de telle manière que la recirculation dans une fistule ($R_A$) peut être déterminée à partir des sommes ($R_H$ et $R_L$), déterminées dans les cas du premier et du deuxième débit sanguin ($Q_{BH}$ et $Q_{BL}$), de la partie de circulation cardio-pulmonaire ($R_{CP}$) et de la recirculation dans une fistule ($R_A$) selon l'équation qui suit :

$$R_A = \frac{(1 - R_L)R_H k - R_L(1 - R_H)}{(1 - R_L)k - R_L(1 - R_H)}$$

où

$$k = \frac{Q_{BL}}{Q_{BH}}.$$

4. Dispositif selon la revendication 1 ou 2, **caractérisé en ce que** l'unité de calcul et d'analyse (18) est réalisée de telle manière que la recirculation dans une fistule ($R_A$) peut être déterminée à partir des sommes ($R_H$ et $R_L$), déterminées dans le cas du premier et du deuxième débit sanguin ($Q_{BH}$ et $Q_{BL}$), de la partie de recirculation cardio-pulmonaire ($R_{CP}$) et de la recirculation dans une fistule ($R_A$) selon l'équation qui suit :

$$k = \frac{Q_{BL}}{Q_{BH}} \cdot (\frac{1}{R_L} - 1)$$

$$R_A = 0,5\left[ R_H + k - \sqrt{(k - R_H)^2 + 4(1 - R_H)} \right]$$

5. Dispositif selon la revendication 3 ou 4, **caractérisé en ce que** l'unité de calcul et d'analyse (18) présente des moyens servant à obtenir la différence entre la somme de la partie de recirculation cardio-pulmonaire ($R_{CP}$) et de la recirculation dans une fistule ($R_A$) d'une part et la recirculation dans une fistule ($R_A$) d'autre part afin de déterminer la partie de recirculation cardio-pulmonaire ($R_{CP}$).

6. Dispositif selon l'une quelconque des revendications 1 à 5, **caractérisé en ce que** le système (20) servant à modifier une grandeur caractéristique physique ou chimique dans le sang est un système servant à faire varier la température (T) du sang.

7. Dispositif selon l'une quelconque des revendications 1 à 6, **caractérisé en ce que** le système (21, 22) servant à mesurer une grandeur caractéristique physique ou chimique dans le sang est un système servant à mesurer la température du sang.

8. Dispositif de traitement du sang comprenant un dialyseur (6) séparé par une membrane (7) semi-perméable en un compartiment de sang (8) et en un compartiment de liquide de dialyse (9), un conduit de sang (10) artériel menant au compartiment de sang du dialyseur et un conduit de sang (11) veineux partant du compartiment de sang d'un circuit de sang extracorporel (I), une pompe à sang (25) étant disposée dans le circuit extracorporel, un conduit d'amenée de liquide de dialyse (13) menant au compartiment de liquide d dialyse (9) et un conduit d'évacuation de liquide de dialyse (14) partant du compartiment de liquide de dialyse (9) du dialyseur (6), **caractérisé par** un dispositif servant à déterminer la recirculation ($R_A$) dans une fistule et/ou la partie de recirculation ($R_{CP}$) cardio-pulmonaire selon l'une quelconque des revendications 1 à 7.

Fig. 1

Fig. 3

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19702441 C1
- DE 19528907C1 A
- US 6537240 B2
- WO 2006072271 A1
- DE 19541783 C1
- DE 19528907 C1

**In der Beschreibung aufgeführte Nicht-Patentliteratur**

- **D. SCHNEDITZ et al.** Cardiopulmonary recirculation during hemodialysis. *Kidney Int.,* 1992, vol. 42, 1450-1456
- **W. BAY et al.** Color Doppler flow predicts PTFE graft failure. *J. Am. Soc. Nephrol.,* 1994, vol. 5, 407
- Models to predict recirculation and its effect on treatment time in single-needle dialysis. **F. GOTCH.** First Intl. Symposium on Single-Needle Dialysis. ISAO Press, 1984, 305
- *EDTNA-ERCA Journal,* 1993, vol. 19, 6
- **DRUKKER ; PARSONS ; MAHER.** Replacement of Renal Function by Dialysis. Kluwer Academic Publishers, 2004
- **SCHNEDITZ et al.** *Seminars in Dialysis,* 2003, vol. 16 (6), 483-487
- *Semin Dial,* 2003, vol. 16 (6), 483-7